# EUROPEAN PATENT APPLICATION

(11) **EP 4 177 604 A1**
(43) Date of publication of application: **10.05.2023**
(21) Application number: 21207259.9
(22) Date of filing: 09.11.2021
(51) Int. Cl.: G01N 33/44

(54) **METHOD AND SYSTEM FOR DETERMINING THE QUALITY OF A CLOSED CELL FOAM WITH RESPECT TO THERMAL INSULATION**

(71) Applicant: Brugg Rohr AG Holding, 5200 Brugg (CH)
(72) Inventor: Kress, Jürgen, 8966 Oberwil-Lieli (CH)
(74) Representative: E. Blum & Co. AG

(57) **Abstract**

The invention relates to a method for determining the quality of a closed cell foam (9) with respect to its thermal insulation, to a system for carrying out the method and to an insulated pipe with such a system. A characteristic parameter of cell gas present in the closed cell foam is measured, wherein the method comprises the steps of introducing a sensor (1) adapted to measure the characteristic parameter into the foam (9), triggering by means of a control and processing device a measurement of the characteristic parameter in the foam by the sensor (1), thereby obtaining a corresponding current measured value which is transmitted to the control and processing device (12), and determining the quality of the closed cell foam (9) by comparing by means of the control and processing device the current measured value or a replacement value derived from the current measured value with a corresponding reference value.

## Description

### Technical Field

The invention relates to a method for determining the quality of a closed cell foam with respect to its thermal insulation, a system for carrying out the method and a thermally insulated pipe according to the independent claims.

### Background Art

Closed cell foams are widely known and applied in various fields such as HVAC (heating ventilation and air conditioning), marine and automotive due to their properties of effectively reducing liquid and gas flow through them and having a high water resistance. Other properties of closed cell foams, which are of central importance for a preferred application of the present invention, are exhibited by the fact that closed cell foams have a low thermal conductivity and are good air barriers and are rigid and durable. Due to these properties, closed cell foams are widely used as insulation materials, e.g. for pipes, insulation boards etc.

Closed cell foams contain so-called cell gases. The heat conductivity, called Lambda value (λ-value) of a closed cell foam, is composed of three components: the λ-value for the radiation, the λ-value of the polymeric matrix, and the λ-value of the cell gases themselves. The latter dominates the overall λ-value of bulk material, a value of about 60-70 % is normally stated (The polyurethanes handbook, Ed David Randall and Steve Lee, John Wiley 2002, ISBN 0-470-85041-8, p. 234). In order to develop foams with a lower heat conductivity it is therefore normally tried to reduce the λ-value of the cell gases. This is e.g. done by adding blowing agents having a low heat conductivity, e.g. hydro fluoro olefins (HFO). A problem with other methods of determination of the heat conductivity of bulk samples is that a piece of the sample has to be cut and in that very moment the cell gases start to diffuse out of the foam and air diffuses into the foam, thus undermining the quality of the measurement itself. Another problem with conventional destructive methods is the fact that for the investigation of the long term changes of the λ-value of a certain foam many samples have to be prepared at the same time so that there are enough samples for destructive testing later on. That process requires much time and material and increases the error of the measurement series because the samples will never be 100 % identical. Another problem with conventional destructive methods is that they can obviously not be applied to a work piece which is in operation, often those parts are part of a fixed installation, e. g. insulation boards at building surfaces or insulated pipes laid underground.

Depending on environmental conditions, closed cell foams may be very durable and ensure a good thermal insulation for decades. This is particularly the case when using closed cell foams as insulation layers comprised in district heating pipes, as such insulation layers are surrounded by other protective layers and a rigid outer jacket. Hence, degradation of the insulation layers are kept to a minimum due to such measures. To date, it has been regarded as sufficient to apply such protective measures due to the high reliability of closed cell foams and degradation has been dealt with punctually only in case of failures detected based on other parameters, such as, exemplarily for insulated pipes, ingression of humidity and therefore a loss in transported energy, or corrosion of other pipe elements. Loss of heat means that the typically low thermal conductivity of the insulation layer increases e.g. at a certain position along the pipe, thereby decreasing the insulation property of the pipe.

There are several methods to determine the heat conductivity of bulk samples of insulating materials, e. g. the heat flash method or heat flow meters (https://www.linseis.com/produkte/waermeleitfaehigkeittemperaturleitfaehigkeit/) or (https://www.netzschthermal-analysis.com/de/produkte-loesungen/waerme-und-temperatur-leitfaehigkeitsbestimmung/).

However, such methods need samples which have to be taken out of the work piece. Hence, these methods are destructive in nature or the corresponding devices are not easy and cheap enough to be applied in large numbers, for example in order to monitor pipe systems of a length of hundreds of meters or insulated boards which are part of a housing fagade.

### Disclosure of the Invention

Hence, it is a general objective of the invention to provide a method of assessing the state of closed cell foams in terms of their insulation quality and facilitating a prediction of possible upcoming failures. Furthermore, it is an objective of the invention to monitor the state of closed cell foams over time and gather information about changes in the foam properties depending on specific environmental factors that the foam is exposed to. Finally, it is an objective of the present invention to provide a pipe which is insulated with a closed cell foam and exhibits the capability of assessing its insulation quality.

Now, in order to implement these and still further objects of the invention, which will become more readily apparent as the description proceeds, in **a first aspect of the invention,** the method for determining the quality of a closed cell foam with respect to its thermal insulation is manifested by the features that a characteristic parameter of cell gas present in the closed cell foam is measured, comprising the steps of:
a) introducing at least one sensor adapted to measure the characteristic parameter into the foam,
b) triggering by means of a control and processing device at least one measurement of the characteristic parameter, in the foam by the sensor, thereby obtaining a corresponding current measured value which is transmitted to the control and processing device,
c) determining the quality of the closed cell foam by comparing by means of the control and processing device the current measured value or a replacement value derived from the current measured value with a corresponding reference value.

It is most preferred that the characteristic parameter is selected from a thermal conductivity and/or a carbon dioxide concentration and that the at least one sensor is selected from a thermal conductivity sensor and/or a carbon dioxide concentration sensor, respectively.

A **second aspect of the invention** relates to a system for carrying out the method according to the first aspect of the invention. The system comprises at least one sensor for measuring the characteristic parameter. Furthermore, the system comprises a control and processing device for triggering measurements by the sensor and for receiving the measurement values from the sensor and for determining the quality of the closed cell foam by comparing at least one measurement value with a maximum prescribed value for the closed cell foam. The maximum prescribed value is a value indicative of the quality of the closed cell foam and will be described later. The sensor comprises a sensing element and electronic circuitry connected to the sensing element. The electronic circuitry is encapsulated in a heat insulating and airtight casing and the sensing element itself is encapsulated in a diffusion-open casing.

There are numerous advantages of assessing the insulation properties of closed cell foams using such sensors as compared to other conventional methods. The cell gas mixture of a closed cell foam makes up most of the total heat conductivity of such a foam. Therefore it is of utmost importance to have a non-destructive method at hand for determining the heat conductivity of the cell gas mixture directly. Such a measurement is fast, reliable and can be repeated at any point in time and allows for the determination of a possible change of the thermal conductivity over time, for example due to aging processes.

Thus, the invention can be used to monitor the heat conductivity of the foam over a prolonged period of time up to the entire life time of the work piece, e. g. an insulated pipe.

Another example is an ingress of other gases into the system. Such diffusion phenomena like a slow ingress of air into the sample over a period of months or even years can be monitored. In such a way it is possible to assess the quality or the effect of diffusion barriers or other technical means which could have an influence on the composition of the cell gas mixture.

Advantageously, the system according to the invention is inexpensive, so it can be used in large numbers, is easy to install, is durable and provides relevant technical data over a prolonged period of time.

The invention can also be used for the detection of a leakage which might occur over time. For example in insulated pipes for district heating, the insulating foam is surrounded by a polyethylene jacket. In case that jacket is damaged during installation and that error was not detected and the pipe is laid in the ground, a change in the λ-value of the cell gas mixture can indicate such a damage afterwards and the respective pipe can be replaced.

In one preferred target application, the system according to the second aspect of the invention is used for determining the quality of a thermal insulation foam comprised in insulating pipes, particularly district heating pipes. The district heating pipes in question preferably satisfy, but are not limited to the standards EN 253, DIN EN 15632-1/2/3/4 in their respective version that is valid at filing date of this document.

In another preferred target application, the system according to the second aspect of the invention is used for determining the quality of a thermal insulation foam comprised in insulating panels, particularly in building wall insulating panels or in building roof insulating panels.

### Brief Description of the Drawings

The invention will be better understood and objects other than those set forth above will become apparent when consideration is given to the following detailed description thereof. Such description refers to the annexed drawings, wherein it is shown in:
Fig. 1 a system according to the invention,
Fig. 2 a cross section view of an arrangement for measurement of thermal conductivity or carbon dioxide concentration according to the invention in a simplified district heating pipe,
Fig. 3 a diagram illustrating measurements carried out with the system according to the present invention in a first use case, and
Fig. 4 a diagram illustrating measurements carried out with the system according to the present invention in a second use case.

### Modes for Carrying Out the Invention

For reasons of simplification, if the term sensor in the following text is used alone, it refers to either one of the thermal conductivity sensor or the CO₂ concentration sensor, except for sections where the type of sensor is relevant.

In the following, the invention is described with reference to its preferred application in insulated district heating pipes but it obviously may be applied in other configurations of closed cell foams.

For the purpose of the present invention, closed cell foams encompass but are not limited to polyurethane (PUR), polyisocyanurate (PIR) and thermoplastic foams such as PET-foams or PE-foams. For the purposes of the present invention, PUR-based or PIR-based foams typically have, but are not limited to a density in the range between 40 - 150 kg/m³, depending on the use case.

The total heat/thermal conductivity (λ-value) of polyurethane foam is e.g. described in detail in the paper "thermal conductivity of polyurethane foam- best performance" within the context of the 10th international symposium on district heating and cooling September 3-5, 2006, section 6a- pipe properties, page 3(11) ff.

It is noted that any mentioned "value" in the context of a comparison is assumed to be of the type of the value it is compared to.

For the purpose of the preferred characteristic parameter, which is the thermal conductivity and/or the CO₂ concentration, the sensor according to the invention may be identical for both parameters. An example for such a sensor by means of which both parameters are obtainable is given below. Therefore, in instances where multiple sensors are used, which will be described in the course of the following text, it is conceivable to use multiple sensors of which one or more may be used for obtaining one of said parameters and the other sensor(s) are used for obtaining the other parameter. However, it is noted that the characteristic parameter is not limited to the two enumerated parameters. For example, humidity may be another valid parameter for the purposes of the invention.

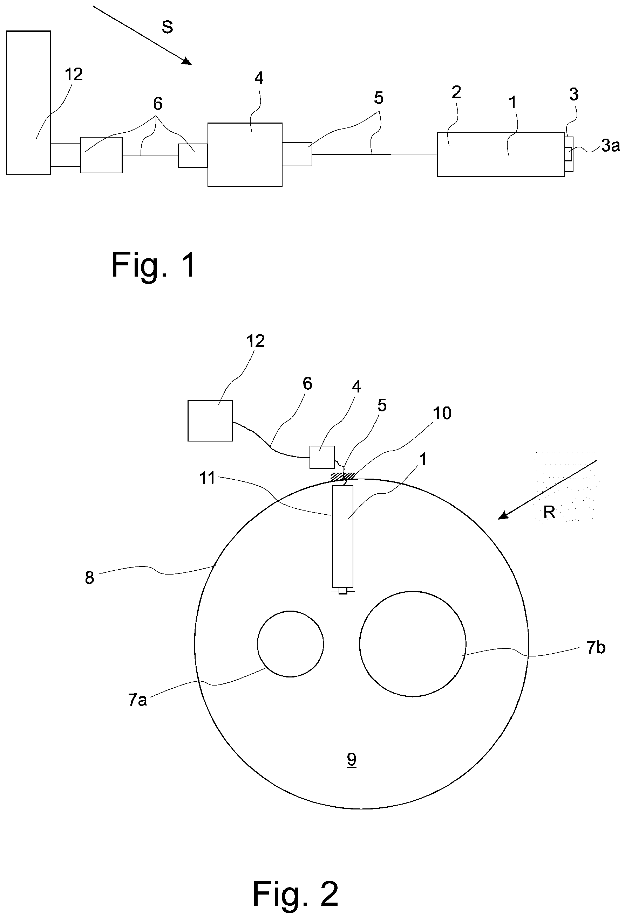

**Fig. 1** shows a system S according to the invention. The system comprises a thermal conductivity sensor or a carbon dioxide concentration sensor 1 for measuring the thermal conductivity or the carbon dioxide concentration, respectively. As mentioned, a single sensor can be chosen, which is configurable to either measure thermal conductivity or CO₂ concentration. Such a sensor is for example available by the company Sensirion, Stäfa, Switzerland (Sensor type STC31). Other sensors may also be used, for example the sensor XEN-3880 by the company Xensor Integration, Ej Delfgauw, Netherlands. As the description proceeds, the measurement of either thermal conductivity or CO₂ concentration and the relevance of the two measurement methods for the invention will be explained in more detail. That sensor has sensing elements for heat conductivity (respectively CO₂ concentration), temperature, and relative humidity.

The sensor comprises a sensing element and electronic circuitry connected to the sensing element. Generally, the electronic circuitry is encapsulated in a casing for protecting it against outer influences. It is preferred that the casing provides mechanical protection, potentially protection against heat, dust or foam particles, etc. This is particularly advantageous if the sensor is introduced into the closed cell foam before the closed cell foam is being formed by reaction of a two component mixture consisting of at least one polyol and at least one isocyanate. It has been found in experiments that the reliability of the sensor decreases significantly if applied without an additional protection casing which considerably reduces the risk of damaging or even entirely destroying the sensor due to mechanical stress. Such casing is described below.

Generally, the sensing element is encapsulated in a diffusion-open casing. Advantageously, this allows gas to be analyzed to access the sensing element but at the same time protects the sensing element from contamination with solid particles like foam particles produced during the insertion of the sensor into the foam or with dust.

In the present example, the sensor 1 is surrounded by a single casing 2, which encompasses both the heat insulating casing and the diffusion-open casing, where the latter is represented in **Fig. 1** by a cap 3 on the front face of the casing. The cap surrounds the sensing section 3a of the sensor. The casing 2 preferably comprises a rigid outer shell designed to be resistant to mechanical stress of the typical order to be expected in the foam. This includes mechanical stress during the foaming process and equally during the hardening process of the foam. The casing 2 may further comprise on the inside at least one insulation layer or tube, which does not obstruct the diffusion-open cap 3.

In embodiments, the sensor 1 may be connected to a bridge 4 by a first cable 5 and the bridge may be connected to a control and processing device 12 for triggering measurements by the sensor 1 and for receiving the measurement values from the sensor 1 by a second cable 6. Preferably, the bridge is configured to allow connection of multiple sensors 1 to the control and processing device 12. But the control and processing device 12 may also be connected to the sensor by means of a single appropriate cable. In either case, the connection may be a USB-connection or another type of suitable connection. Other types of suitable connection may also be used.

The control and processing device 12 is also adapted for determining the quality of the closed cell foam by comparing at least one measurement value with a maximum prescribed value for the closed cell foam. Typically, the control and processing device 12 is a computer or an ASIC integrated in a computer. In this context, it is noted that the system S may be a distributed system, that is: one or multiple parts of the system S may be located inside a thermally insulated pipe or another workpiece, like an insulating panel, and communicate with the rest of the parts which may be located outside the thermally insulated pipe. This will become apparent from the description in connection with Fig. 2.

In embodiments, the sensor is chosen to operate according to either one of different measurement methods including but not limited to: transient hot wire measurement method or transient line source method or non-dispersive infrared measurement method.

The transient hot wire measurement method is used to measure the thermal conductivity by measuring a temperature gradient at a certain distance from a heated element. A description of this method can be found at https://www.tec-science.com/thermodynamics/heat/transient-hot-wire-method-method-for-determining-thermal-conductivity-thw. The advantage is that the measurements are fast as compared to other methods.

The transient line source method is used to measure the thermal conductivity by measuring a temperature gradient, where a suitable sensor and a heated element are arranged in a same rod. A description of this method can be found at https://thermtest.com/tls-100. An advantage of this method is the small size of the sensor arrangement as compared to other methods, while yielding a measurement result within ca. 1 min.

The non-dispersive infrared measurement method is used for measuring light transmission in the infrared range and deriving the CO2 concentration from the transmitted light. A description of this method can be found at https://jlcinternational.com/co2-measurement-theory-basics. This method allows measuring the CO2 concentration, from which the thermal conductivity can be derived.

In the following, the steps of the method according to the invention will be explained in more detail along with their individual optional features.

The step a) of introducing the sensor into the foam may be carried out in the following two ways with a number of individual substeps, respectively.

A first option is to introduce the sensor into the foam before a foaming process. Firstly, a heat protection element can be applied to the sensor if the sensor is not already encapsulated in a heat protection casing, as described above. Subsequently, the thermal conductivity sensor or the carbon dioxide concentration sensor, respectively, is attached in its intended position in a space to be foamed. This may be accomplished in a simple way by hanging the sensor in the space to be foamed by means of a stiff metal tube to which the sensor is firmly attached and which is capable of holding the sensor in place when a force originating from the pressure created by the foaming device acts on the sensor. The tube is preferably adapted in such a way that it also guides the connection cable of the sensor to the outside of the space to be foamed and also protects said cable from the hot foam. Finally, the foam is applied in said space and is left to harden. Thus, the sensor is permanently arranged inside the foam and is tightly encapsulated by the hardened foam. This option has the advantage, again based on the present example of district heating, but which applies mutatis mutandis to other applications, that the district heating pipe can be prepared with analysis means directly during manufacturing and can be delivered in a state ready to be monitored.

In connection with **Fig. 2****,** a second option is to introduce the sensor 1 after the foam has been applied and has hardened. Fig. 2 shows a cross-section of an exemplary thermally insulated pipe R as a district heating pipe. The pipe R has two medium pipes 7a, 7b with different diameters for transporting one or two fluids. Of course, only one medium pipe or more medium pipes are also possible. The pipe R further comprises an outer jacket 8 for protecting the thermally insulated pipe R and a thermal insulation 9 which fills a space between the medium pipes 7a, 7b and the outer jacket. A cavity 11 is provided through the outer pipe, which reaches into the thermal insulation down to an intended measurement position. This is done by drilling a hole into the hardened foam 9 down to an intended position of the sensor and introducing the sensor 1 into the hole 11 in the intended position. The gap between the sensor and the pipe can be sealed with an appropriate glue in order to fix the sensor permanently in its position and to prevent gas diffusion from the outside environment towards the sensing section of the sensor.

In this option, the sensor may either be permanently left in the foam or may be removed after a measurement or a series of measurements have been executed. This has the advantage that the sensor is only required where needed and therefore this option is less costly as compared with the first option, particularly for the present example in view of the fact that district heating pipes may be tens of kilometers long. A further advantage of this option is that the foam is per default kept more homogenous by not having alien elements encapsulated inside it. However, it is more time consuming to prepare an insulated district heating pipe with one or more sensors in this way.

For the second option it is preferred that an intermediary step of sealing the hole 11 with the sensor is carried out before step b) in order to prevent an influence of an environment atmosphere on the measurements. This is achieved by means of a seal 10 which seals the cavity 11 in an air-tight and liquid-tight way. As mentioned above, environmental air is a detrimental factor because it falsifies the CO₂ measurement or the thermal conductivity measurement. The hole is sealed preferably with a fast hardening resin-based glue, which is particularly preferred if the sensor shall be left inside the foam permanently. However, other types of sealing materials may also be used, particularly sealing materials that are easily removable in case the sensor shall be removed after a series of measurements.

For the preferred application in thermally insulated district pipes, the system according to the invention is used along the pipe length but it may also be used at connection points between pipe segments. This is advantageous because the assessment of insulation quality in such joints or sleeves or fittings is particularly informative of potential faults in these sensitive areas. For this purpose, the thermally insulated pipe further comprises at least one connection sleeve for connecting the pipe with a further pipe, wherein the connection sleeve is attached to one end of the pipe, wherein the connection sleeve comprises an insulation layer having incorporated therein at least one additional sensor for measuring the characteristic parameter. This may provide an indication of potential corrosion in metal parts of the sleeve, e.g. at the welding points between two inner pipe segments when the insulated pipe contains an inner metal fluid pipe. Such sleeves that may be equipped with the system according to the invention are e.g. the Brugg Visucon connection sleeve or the Brugg CALPEX Big I-shell.

The sensor 1 is equipped with a transmission and reception device (not shown) adapted to communicate with the control and processing device 12 of the system according to claim 11 or 12, through the seal.

It is generally preferred to carry out a series of measurements and not a single measurement, thereby obtaining a curve of the measured value over time. Advantageously, even small value variations can be detected over time in this way. In view of this, when step b) is carried out repeatedly, there are a number of options:
The measurements may be carried out on-demand by manually triggering each measurement or a series of measurements via the control and processing device. This option is particularly advantageous when the state of the insulating foam shall be monitored over a long period of time, particularly in order to gather data about long term changes in the thermal insulation of the pipe. Such data may advantageously be used to analyse a long term degradation (for example during years or decades) of the insulation in view of specific parameters which are unique to the certain pipe and its surroundings. For this purpose it is not necessary to perform continuous measurements which would yield huge amounts of data. As mentioned, there is the suboption to trigger one measurement or a series of measurements for a limited time period, which is related to the next possibility.

It is possible to carry out measurements automatically. This can be done in one alternative by specifying a measurement pattern containing a set of measurements to be executed during a certain period of time and triggering each measurement at a corresponding instant during said certain period of time. This possibility is advantageous if multiple measurements shall be performed, however not periodically. A scenario for this alternative would be the triggering of measurements based e.g. on external criteria. Such criteria may be data from other sensors, for example sensors monitoring environment temperature. Thus, the control and processing device may be configured to accept different patterns of trigger conditions, either programmable patterns or patterns based on an automatic input of said other sensor data to the control and processing device, for the purpose of which the control and processing device is equipped with the necessary interfaces for the other sensors. This is advantageous due to the flexibility of measurements.

In another alternative, the automatic measurements can be performed by triggering a measurement periodically during a certain period of time with a desired measurement frequency. This is advantageously a simple way of gathering a series of measurement data with little programming effort.

In case the measurements are triggered automatically, said step b) may be terminated when a termination condition is fulfilled.

The termination condition may be activated when a certain period of time has passed. This is particularly advantageous if the operator knows that thermal balance is present inside the foam.

Alternatively or additionally, the termination condition may be activated if a calculation of a deviation of the current measured value from at least one previous measured value always yields a result below a specified value for a predetermined number of calculations. This option is particularly advantageous if the operator is not sure if thermal balance and/or a stable CO₂ concentration are/is present inside the foam. As mentioned, reaching thermal balance may take a long time depending on a variety of factors. However, the measurement results can only be considered valid when the CO₂ concentration is stable and advantageously in the state of thermal balance. Even more important is the fact that it may take an even longer time until the cell gas from all parts of the foam around the sensor has reached the sensor and the air which was introduced into the hole when the sensor was initially inserted has diffused into the foam. This option accounts exactly for this scenario. It has been observed that certain curve types occur while the immediate measurement environment is not yet thermally balanced, which curves show large gradients between consecutive measurements. As soon as thermal balance is present, said gradients decrease. This fact can be used to determine, particularly based on empirical values, when the gradient between two consecutive measurements can be considered low enough to validate a measurement. Preferably, the termination condition is not activated after a single gradient value has been acknowledged as valid but after a series of gradients show similar values, in order to account for a possible single erroneous measurement yielding such false "valid gradient".

It is noted that the entire description of series of measurements may also be applied by using average values calculated from subsets of measurement values rather than absolute single measurement values.

In case the carbon dioxide concentration of cell gas is measured, the step c) is preferably carried out either by comparing the current measured value directly with a prescribed carbon dioxide concentration value representing said reference value or by first deriving from the current measured value of the carbon dioxide concentration a thermal conductivity value representing the replacement value mentioned at the beginning, and subsequently comparing the thermal conductivity value with said corresponding reference value. The first alternative accounts for cases when a prescribed carbon dioxide concentration value has been calculated in advance by performing tests yielding the correlation of thermal conductivity with CO2 concentration. In such cases a direct comparison is enough and simplifies the process. The second alternative accounts for situations in which no experience values relating to the correlation between CO₂ concentration and thermal conductivity are available. Therefore, after preforming the CO2 measurements, an additional step of deriving the thermal conductivity from the CO₂ measurements is carried out, which yields said replacement value. In this context it is noted that the term "replacement value" is understood in general as a value used for the quality assessment which was not directly measured but derived from a directly measured value.

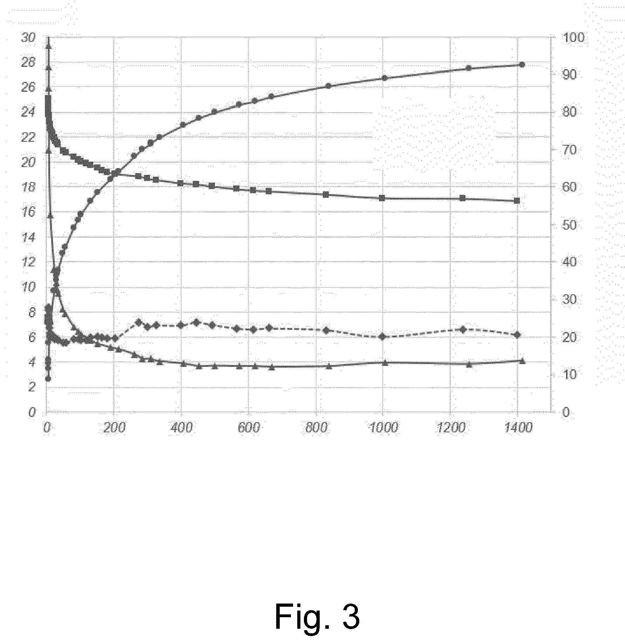

In the following, a practical arrangement illustrating measurement results achieved with the system according to the invention is given as a first example with reference to the already described figures and to **Fig. 3****.**

A sensor 1 of the type STC31 from the company Sensirion, Stäfa, Switzerland is connected via an USB-cable 5 to the sensor bridge 4 (USB bridge) and the bridge is connected via its USB connection 6 to a computer, which comprises the control and processing device 12. The sensor is placed in a metal tube and glued into that tube with a two component epoxy resin in such a way that only the front end of the cap 3 carrying the sensing section 3a remains uncovered by the glue but remains in the space of the metal tube. This front end is closed with a lid having a small hole drilled into it. The lid is covered with a piece of porous fabric or fleece, e. g. a layer of a surgical mask which is fixed on the metal tube with an adhesive tape in such a way that the adhesive tape does not cover the hole drilled in the lid.

The above system is now used in a practical measurement arrangement which shall simulate the measurement conditions in a real thermally insulated district heating pipe.

Firstly, a thermal insulation is produced. For this purpose, an amount of 78 g of the polyol Daltofoam TE34201 available from the company Huntsman, which was premixed with 6 g of cyclopentane and 7.3 g of the physical blowing agent Solstice LBA from the company Honeywell, is placed into a metal bucket with a height of 200 mm and an outer diameter of 160 mm, available from the company Kop, 8583 Sulgen, Switzerland. A quantity of 120 g of a polymeric isocyanate type Suprasec 5025 (company Huntsman) is added all at once. The mixture is intensively stirred with a mechanical stirrer for ten seconds. After the foam has reacted and cooled down after about 20 minutes, the thermal insulation is ready and hardened. The excess foam protruding out of the metal bucket was removed with a saw and the opening is covered with an appropriate metal plate and fixed with a two-component epoxy resin. A hole is drilled into the hardened foam at the centre of the bucket and the sensor of the system described above is inserted into the hole in the foam and the gap between the metal tube of the sensor package and the bucket is sealed with a seal made of a two component epoxy resin type EA3475 from the company Loctite. This particular resin type is especially preferred because it minimizes gas diffusion from outside. Subsequently, a measurement series was carried out with the system. A plot illustrating the measurement according to the first example is shown in Fig. 3.

The left vertical axis in Fig. 3 denotes the λ-value in mW/m*K, and relative humidity in percent, as the case may be, the right vertical axis denotes the apparent CO₂ concentration in volume percent and the temperature in °C, as the case may be. The horizontal axis denotes time in hours. The curves represent the respective variable as indicated in the diagram, that is:
- the curve with squares represents the heat conductivity over time (left vertical axis),
- the curve with the triangles denotes the relative humidity in percent (left vertical axis),
- the curve with the circles represents the CO₂ concentration in percent (right vertical axis), and
- the dashed curve with the rhombs denotes the temperature in °C (right vertical axis).

As can be seen in the diagram, the heat conductivity starts at the level of about 26 mW/m*K because of the outside air introduced into the hole during insertion of the sensor package. The heat conductivity drops over time, as does the relative humidity. The apparent CO₂-concentration is increasing. The explanation for that is as follows:
The sensor STC31 is commercially available and used for the detection of the CO₂ concentration in air. Air has a λ-value of 26 mW/m*K, CO₂ has a λ-value of 16 mW/m*K. With respect to that setting, a decreasing heat conductivity is interpreted as an increasing CO₂ concentration because only CO₂ with its low heat conductivity can explain that outcome within the logical framework of the algorithm. In contrast to such experiment arrangement in ambient air, in a closed cell foam other gases are present, which have a lower λ-value than CO₂. For example cyclopentane has a λ-value of 13 mW/m*K. Therefore, the total λ-value of the cell gases is considerably lower than air. For the CO₂ sensing system this is then interpreted as an increasing CO₂ concentration.

It can be seen from the diagram of Fig. 3 that a long time is needed until a stable value for the heat conductivity and the CO₂ concentration has been reached. As mentioned above, this is due to the fact that a small amount of air is introduced into the system when the sensor is inserted into the hole in the foam.

Now, if the apparent CO₂ concentration shall be measured and a conclusion regarding the heat conductivity of the cell gas shall be drawn therefrom, the procedure outlined below is preferred and most straightforward. This procedure can be regarded as a calibration process of the CO₂ concentration sensor and is preferably carried out at an arbitrary time before step a).

The procedure comprises the step of preparing the foam of interest and which is representative for the real end product. That means in other words a preparation of the foam with all the ingredients and in the same amounts as used for the real end product. The preparation of the foam in this way is important because a foam is made up of several components like polyol, isocyanate, a defined amount of water in the polyol, physical blowing agents like cyclopentane of HFOs and so on, such that measurement results may significantly vary in case the intended foam composition is not observed. With such a sample foam, the heat conductivity and the CO₂ are measured at the same time in parallel and for an extended period of several hours or a couple of days. A plot of the measured values yields curves similar to the exemplary curves of Fig. 3, where each value for the apparent CO₂ concentration corresponds to a value of the heat conductivity. The measured curves are then stored. With this data, it is sufficient for future applications to measure only the apparent CO₂ concentration in order to obtain a value for the heat conductivity by reading out the corresponding heat conductivity value from the stored curves.

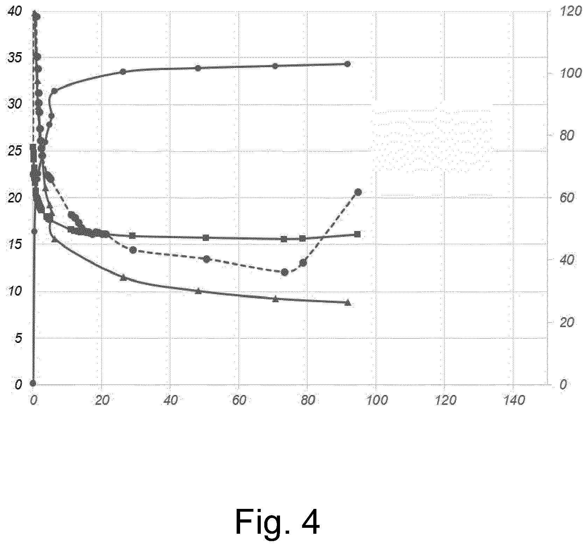

The above procedure can also be used for multiple foam samples with varying concentrations of the components as a concentration series. **Fig. 4** shows a diagram illustrating measurements carried out with the system according to the present invention in a second use case. The second use case reflects the introduction of the sensor into the intended foaming space before the foaming process, in contrast to the first use case, where a hole was drilled into the already hardened foam and the sensor was introduced in that hole.

The practical arrangement for the second use case is described as a second example in the following. A hole is drilled in the center of an aluminium pipe, length = 184 mm, wall thickness = 5 mm, inner diameter = 170 mm. A prefabricated sensor according to the first example is placed into the hole and fixed with epoxy resin type EA3475. The bottom of the aluminium pipe is closed with a round piece of metal which is just slightly larger in diameter than the pipe itself. It is permanently fixed with epoxy resin. A thermal insulation is produced. For this purpose, 24 g of cyclopentane are dissolved in an amount of 400 g of the polyol Daltofoam TE34201. Of that mixture, 92.2 g are weighed into the prefabricated aluminium pipe. An amount of 171.5 g Suprasec 5025 is added while the mixture is being stirred for 10 seconds. After the foam has been formed and hardened, the excess foam protruding out of the aluminium pipe was removed with a saw. The top was covered with a metal plate like in case of the bottom part and sealed off with epoxy resin. The measurements of the relative humidity, the apparent CO₂ content, the temperature, and the heat conductivity were initiated before the foam was formed and continued for ca. 90 hours. A plot illustrating the measurement according to the second example is shown in Fig. 4.

As in case of Fig. 3, the left vertical axis in Fig. 4 denotes the λ-value in mW/m*K and relative humidity in percent, as the case may be, the right vertical axis denotes the apparent CO₂ concentration in volume percent and the temperature in °C, as the case may be. The horizontal axis denotes time in hours. The curves also represent the respective variable as indicated in the diagram, that is:
- the curve with squares represents the heat conductivity over time (left vertical axis),
- the curve with the triangles denotes the relative humidity in percent (left vertical axis),
- the curve with the circles represents the CO₂ concentration in percent (right vertical axis), and
- the dashed curve with the rhombs denotes the temperature in °C (right vertical axis).

In the diagram, the time interval at the beginning of the measurements until the foam hardened is negligible, typically of about 10 minutes. After that time the foam is still warm but foam expansion has come to a halt. As can be seen in Fig. 4, the CO₂ concentration curve and the thermal conductivity curve exhibit a steep gradient whereafter they stabilize. When compared with Fig. 3, it becomes apparent that this gradient is considerably steeper than in Fig. 3.

This proves that in otherwise same or very similar conditions of the two example arrangements, the time required for reaching stable conditions for the thermal conductivity and the CO₂ concentration is considerably longer when the sensor was introduced after the foam was hardened. This confirms that the amount of air inevitably introduced when inserting the sensor into the hole plays a not negligible role for the time required until valid measurements can be obtained. This conclusion reveals very well the advantage of the alternative in which the termination condition for the measurements may be activated if a calculation of a deviation of the current measured value from at least one previous measured value always yields a result below a specified value for a predetermined number of calculations. In this way, it is thus possible to account for such different time intervals as shown by the comparison of the first and the second example.

In the present invention, it has been discovered that thermal conductivity sensors or CO₂ concentration sensors can surprisingly also be used to obtain valid measurements in closed cell foams, despite difficult measurement conditions. As mentioned above, the available devices containing such sensors are used for analysing gases in thermal balance. However, in closed cell foams, thermal balance is difficult to reach due to a variety of factors: geometry of the foam to be measured, structure of the closed cell system like cell size, influence of environment air, diffusion processes into and out of the cell foam. Therefore, a stable CO2 concentration around the sensor and thermal balance is sometimes only reached after days, weeks or months, depending on the factors enumerated above and additionally on the fact that the introduction of the sensor itself into the closed cell foam may add thermal imbalance due to air inflow into the foam.

Furthermore, it is advantageous that such commercially available sensors, which are designed for the specific use case of detecting the CO₂ concentration in air, can surprisingly also be used for the present application even though in closed cell foams other gases and components are present. Depending on the specific foam composition for the thermal insulation, there are amine-based catalysts, water, physical blowing agents like cyclopentane or hydrofluoro olefins (HFO) and other chemicals to which the sensing unit of the sensor is exposed. In the light of that, it is surprising that the measurements can be carried out with valid results and that the sensor tolerates such conditions.

While there are shown and described presently preferred embodiments of the invention, it is to be distinctly understood that the invention is not limited thereto but may be otherwise variously embodied and practiced within the scope of the following claims. Therefore, terms like "preferred" or "in particular" or "particularly" or "advantageously", etc. signify optional and exemplary embodiments only.

## Claims

1. Method for determining the quality of a closed cell foam with respect to its thermal insulation, wherein a characteristic parameter of cell gas present in the closed cell foam is measured, comprising the steps of:
a) introducing at least one sensor adapted to measure the characteristic parameter into the foam,
b) triggering by means of a control and processing device at least one measurement of the characteristic parameter by the sensor, thereby obtaining a corresponding current measured value which is transmitted to the control and processing device,
c) determining the quality of the closed cell foam by comparing by means of the control and processing device the current measured value or a replacement value derived from the current measured value with a corresponding reference value.

2. Method according to claim 1, wherein the characteristic parameter is selected from a thermal conductivity and/or a carbon dioxide concentration and the at least one sensor is selected from a thermal conductivity sensor and/or a carbon dioxide concentration sensor, respectively.

3. Method according to claim 2, wherein, in case the carbon dioxide concentration of cell gas is measured, the current measured value and the corresponding reference value are CO2 concentration values, wherein the step c) is carried out either by comparing the current measured value directly with a prescribed carbon dioxide concentration value representing said reference value or, if said corresponding reference value is a thermal conductivity value, by first deriving from the current measured value of the carbon dioxide concentration a thermal conductivity value representing the replacement value, and subsequently comparing the replacement value with said corresponding reference value.

4. Method according to claim 2 or 3, wherein the thermal conductivity sensor or the carbon dioxide concentration sensor, respectively, is chosen to operate according to transient hot wire measurement method or according to transient line source method or according to non-dispersive infrared measurement method.

5. Method according to one of the preceding claims 2 to 4, wherein said step a) is carried out
either before a foaming process by first applying a heat protection element to the thermal conductivity sensor or the carbon dioxide concentration sensor, respectively, subsequently attaching the thermal conductivity sensor or the carbon dioxide concentration sensor, respectively, in its intended position in a space to be foamed and finally applying the foam in said space, such that the thermal conductivity sensor or the carbon dioxide concentration sensor, respectively, is permanently arranged inside the foam, or
after the foam has been applied and has hardened by drilling a hole into the hardened foam down to an intended position of the thermal conductivity sensor or the carbon dioxide concentration sensor, respectively, and introducing the thermal conductivity sensor or the carbon dioxide concentration sensor, respectively, into the hole in the intended position, such that the thermal conductivity sensor or the carbon dioxide concentration sensor, respectively, is permanently or removably arranged inside the foam.

6. Method according to claim 5, wherein, in case the thermal conductivity sensor or the carbon dioxide concentration sensor, respectively, is introduced into the foam after the foam has been applied and has hardened, an intermediary step of sealing the hole with the thermal conductivity sensor or the carbon dioxide concentration sensor, respectively, therein is carried out before step b) in order to prevent an influence of an environment atmosphere on the measurements.

7. Method according to one of the preceding claims 2 to 6, wherein the step b) is carried out repeatedly, either
on-demand by manually triggering each measurement or
automatically by either
specifying a measurement pattern containing a set of measurements to be executed during a certain period of time and triggering each measurement at a corresponding instant during said certain period of time, or
by triggering a measurement periodically during a certain period of time with a desired measurement frequency.

8. Method according to claim 7, wherein, in case the measurements are triggered automatically, said step b) is terminated when a termination condition is fulfilled, wherein the termination condition is fulfilled
when said certain period of time has passed, and/or
if a calculation of a deviation of the current measured value from at least one previous measured value always yields a result below a specified value for a predetermined number of calculations.

9. Method according to one of the preceding claims 2 to 8, wherein said step c) is carried out by comparing an average value calculated from a set of measured values or a replacement average value derived from the average value with a corresponding reference value.

10. Method according to one of the preceding claims 2 to 9, wherein, in case the carbon dioxide concentration of cell gas is measured, a calibration process of the carbon dioxide concentration sensor is carried out before step a) by carrying out measurements of the CO2 concentration and simultaneously of the heat conductivity in a calibration foam with known components, which calibration foam is similar to said closed cell foam, thereby obtaining a distribution of values over time, in which distribution each measured CO2 concentration value at a given time corresponds to a measured heat conductivity value and storing the distribution as reference curves.

11. System for carrying out the method according to one of the preceding claims, comprising
at least one sensor adapted to measure the characteristic parameter,
a control and processing device for triggering measurements by the sensor, and for receiving the measurement values from the sensor and for determining the quality of the closed cell foam by comparing at least one measurement value with a maximum prescribed value for the closed cell foam,
wherein the sensor comprises a sensing element and electronic circuitry connected to the sensing element,
wherein the electronic circuitry is encapsulated in a heat insulating, and particularly airtight, casing and
wherein the sensing element is encapsulated in a diffusion-open casing.

12. System according to claim 11, wherein the at least one sensor is a thermal conductivity sensor and/or a carbon dioxide concentration sensor.

13. System according to claim 11 or 12, wherein the diffusion-open casing of the sensing element is adapted to protect the sensing element from particle contamination present in the cell gas, particularly from dust particles and/or loose foam particles and/or water particles.

14. Thermally insulated pipe (R) for transporting a fluid, comprising
at least one medium pipe (7a, 7b) for transporting the fluid,
an outer jacket 8 for protecting the thermally insulated pipe (R),
a thermal insulation (9) which fills a space between the at least one medium pipe (7a, 7b) and the outer jacket,
at least a cavity (11) through the outer pipe into the thermal insulation down to an intended measurement position,
at least one sensor (1) of the system according to claim 11 to 13 arranged inside the at least one cavity,
wherein the cavity is sealed by means of a seal (10) in an air-tight and liquid-tight way from an environment of the thermally insulated pipe so as to protect the sensor from environmental influences,
wherein the sensor, respectively, is equipped with a transmission and reception device adapted to communicate with the control and processing device of the system according to one of the claims 11 to 13, through the seal.

15. Thermally insulated pipe according to claim 14, wherein the thermal insulation (9) is chosen for the group comprising polyurethane foams (PUR), polyisocyanurate foams (PIR), thermoplastic foams, particularly PET-foams.

16. Thermally insulated pipe according to claim 14 or 15, further comprising at least one connection sleeve for connecting the pipe with a further pipe, wherein the connection sleeve is attached to one end of the pipe, wherein the connection sleeve comprises an insulation layer having incorporated therein at least one additional sensor for measuring the characteristic parameter.

17. Use of the system according to one of the claims 11 to 13 for determining the quality of a thermal insulation foam comprised in insulating pipes, particularly district heating pipes.

18. Use of the system according to one of the claims 11 to 13 for determining the quality of a thermal insulation foam comprised in insulating panels, particularly in building wall insulating panels or in building roof insulating panels.
